# EUROPEAN PATENT SPECIFICATION

(11) **EP 0 260 953 B1**
(45) Date of publication and mention of the grant of the patent: **30.03.1994**
(21) Application number: 87308198.8
(22) Date of filing: 16.09.1987
(51) Int. Cl.: A61B 17/34, A61B 8/00

(54) **Device for use in the cannulation of blood vessels**
Vorrichtung für Anwendung in der Kanalisation von Blutgefässen
Dispositif destiné à être utilisé dans la canalisation de vaisseaux sanguins

(30) Priority: 18.09.1986 US 908556
(43) Date of publication of application: 23.03.1988
(73) Proprietor: Yock, Paul G., San Francisco, CA 94127 (US); Selfridge, Alan R., Palo Alto California 94301 (US)
(72) Inventor: Yock, Paul G., San Francisco, CA 94127 (US); Selfridge, Alan R., Palo Alto California 94301 (US)
(74) Representative: Alexander, Thomas Bruce

(56) References cited:
- EP-A- 0 217 689
- WO-A-86/04225
- FR-A- 2 252 582
- FR-A- 2 555 432
- GB-A- 2 157 828
- US-A- 3 556 079
- US-A- 4 249 539

## Description

This invention relates to apparatus for use in the cannulation of blood vessels.

Insertion of arterial and venous catheters for angiography and acute care of patients is a major source of discomfort, morbidity, and even mortality. The problem of accurate location and penetration of arteries and veins is especially acute for patients who may be obese or present an unusual anatomy and who are undergoing cardiac catherization and other radiological procedures such as cerebral angiograms.

The potential utility of Doppler ultrasound for accurately guiding a needle into a vessel has been recognised. Most applications utilize the transmission of ultrasonic waves through the needle and reception of ultrasonic echos by a separate transducer located on the body of the patient and separate from the syringe and needle. Such applications obviously have limited accuracy. U.S. Patent Specification No. 3,556,079 entitled "Method of Puncturing a Medical Instrument Under Guidance of Ultrasound" discloses in one embodiment the placement of both transmitting and receiving transducers in the needle and syringe. Such an embodiment, however, requires a special catheter construction and can give an erroneous signal when the needle engages the blood vessel before penetrating the vessel.

The invention provides an apparatus for use in the cannulation of blood vessels comprising: a hollow needle with an inner lumen, a sharp distal end which is adapted to be inserted into a blood vessel, and a proximal end which is adapted to be secured to a syringe; an elongate stylet which is positioned within the inner lumen of the needle, the stylet comprising an elongate support means, a transducer attached to the support means at the distal end of the stylet for transmitting and receiving ultrasonic waves through the sharp end of the needle and adapted to generate a signal representing the intensity of the received ultrasonic waves, the transducer having front and back faces; and a syringe which is detachably secured to the proximal end of the needle, characterised in that the stylet is spaced from the interior of the needle to facilitate back flow of blood when a blood vessel is pentrated, and by the provision of electrical conductors which are associated with the stylet for transmitting electrical signals to and from the transducer and include a first electrical conductor which extends through the stylet and is electrically connected to the back face of the transducer, and a second electrical conductor which is electrically insulated from the first conductor, extends along the outside of the stylet and is electrically connected to the front face of the transducer.

In the accompanying drawings:
Figure 1 is a schematic representation of a needle being inserted into tissue for cannulation of a blood vessel;
Figure 2 is a plot of Doppler signal intensity versus distance in tissue of the needle in Figure 1;
Figure 3 is a perspective view illustrating a cannulation device;
Figure 4 is a section view of one needle portion and stylet portion; and
Figure 5 and Figure 6 are section views of portions of different stylets.

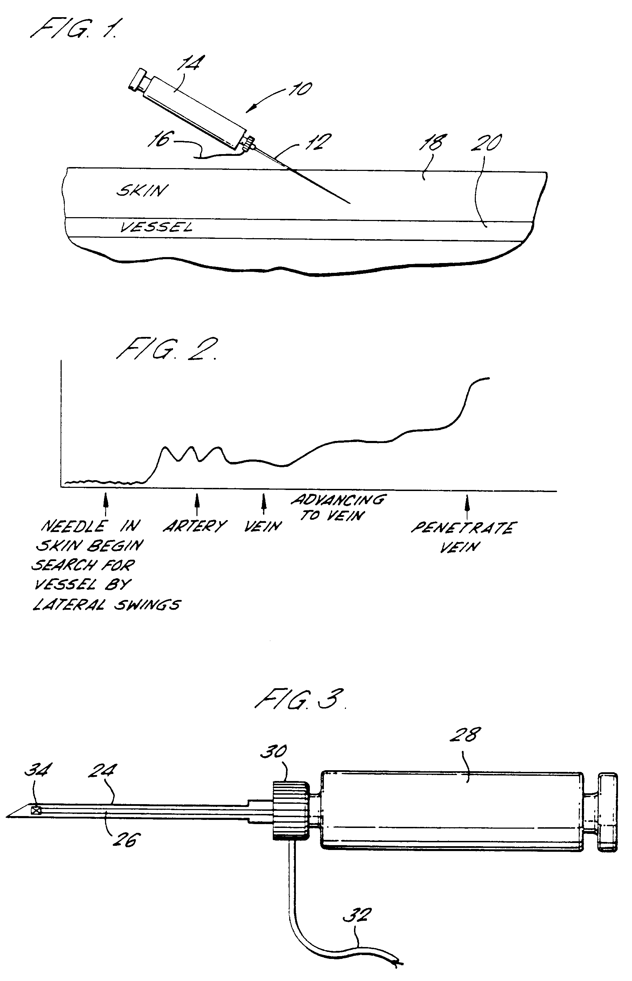

Figure 1 is a schematic illustration of a needle and syringe assembly 10. The assembly 10 includes a needle 12 and a syringe portion 14 with an ultrasonic transducer within the syringe portion 14. Wires 16 are electrically connected with the transducer for the transmission and reception of electrical signals. In the drawing, the needle 12 is being inserted through skin tissue 18 towards a blood vessel 20.

As the needle 12 is passed through the tissue 18, the tip of the needle is moved in a slight arc for directing ultrasound energy transmitted through the needle to the vessel 20. The returned echo signal is used for accurately guiding the needle 12 to the vessel 20 and may provide an indication of when the needle penetrates the vessel 20.

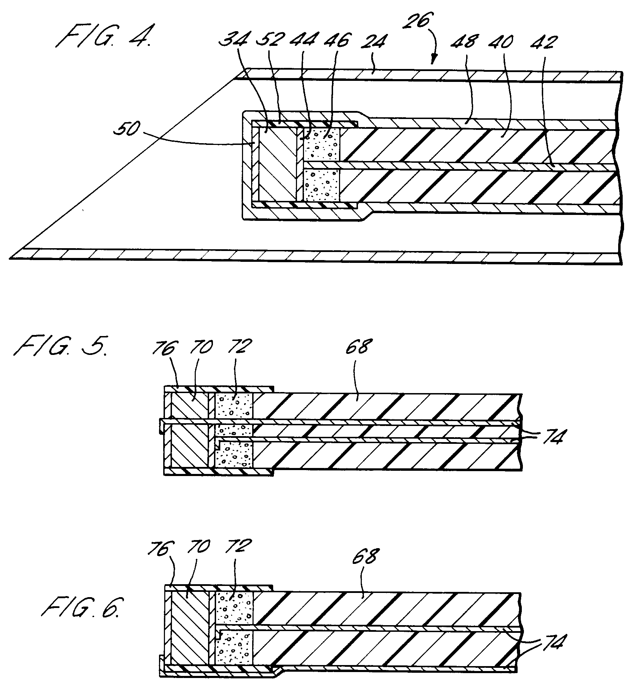

Figure 2 is a plot of the intensity of the Doppler signal against the depth within the tissue 18. When the needle 12 is first inserted into the tissue 18 but not directly towards an artery or vein, the response is small and relatively flat as indicated. Upon pointing the needle 12 at an artery an increased modulated wave is detected; conversely, when the needle is pointed towards a vein, an increased generally uniform signal is indicated. Actual penetration of the vessel will be indicated by the back flow of blood when the vessel is penetrated by maintaining a negative pressure in the needle and a constant back pressure on the syringe while the needle is being advanced. Once the vessel is penetrated, brisk backflow of blood in the needle indicates safe penetration of the vessel and can cause the stepped increase in reflected wave intensity thereby indicating a safe location for injection of medications or passage of a wire into the vessel.

Figure 3 shows apparatus for use in the cannulation of blood vessels which includes a needle portion 24, shown in section view to illustrate a stylet 26 therein. The needle and stylet are connected to a syringe 28 by means of a connector 30. Electrical wires 32 are connected through the stylet with an ultrasonic transducer 34 at one end of the stylet. The transducer 34 is positioned at the sharp end of the needle 24 for the transmission and reception of ultrasonic energy through the open end of the needle.

Figure 4 is a section view of a portion of the needle 24 and the stylet 26 further illustrating the construction of the stylet. The stylet 26 includes a plastics support rod 40 through which a first electrical conductor 42 extends into contact with an electrode 44 on the back face of the transducer 34. The transducer 34 is affixed to the support rod 40 by means of a low acoustic impedance epoxy 46 which is filled with glass microballoons. A second electrical conductor 48 is formed on the exterior surface of the support rod 40 by means of metal deposition and extends into contact with an electrode 50 on the front face of the transducer 34. An insulative material 52 such as an epoxy is formed around the periphery of the transducer 34 to electrically isolate the electrode 44 on the back face from the conductor 48 connected to the electrode 50 on the front face. The transducer 34 is positioned near the sharp end of the needle 24 for the transmission and reception of acoustic energy through the opening in the needle.

In Figure 4, the stylet 26 has an outer diameter less than the inner diameter of the needle 24 whereby blood flow, upon penetration of a blood vessel, is accommodated around the stylet.

In Figure 5 a support rod 68 supports a transducer 70 and a backing epoxy 72. Two separate conductors 74 extend through the support rod 68 with one conductor contacting the back electrode of the transducer 70 and the other conductor extending through the transducer and contacting the front conductor. Where the second conductor passes through the transducer 70, insulation must be provided to electrically insulate the conductor. Again, insulation 76 is provided on the outer surface of the transducer 70, backing material 72 and support rod 68 to electrically isolate the back electrode from the front electrode.

Figure 6 shows a construction similar to the construction of Figure 5, and the same reference numerals are applied to like elements. One conductor 74 extends through the support rod 68 and the backing material 72 to contact the back electrode of the transducer 70. The other conductor 74 is plated on the exterior surface of the support rod 68 and contacts the front electrode of the transducer 70.

In one construction the following materials were employed:
Support rod - 18 gauge stainless steel tubing.
Backing material - Emerson IG0101 microballoons in epoxy.
Transducer - PETSA, 1mm diameter, 20 MHz.
Insulation Material - Ablestix 931-1 epoxy.
Electrical Conductive Material - Trabond 2902 silver epoxy.

The apparatus for use in the cannulation of blood vessels as described above is readily utilised in the Seldinger technique for blood vessel cannulation. After the needle is inserted and guided to a vein by the Doppler technique, penetration of the vein is indicated by the back flow of blood through the needle to the syringe portion. The syringe is then removed from the needle and the connector, and the stylet is then removed from the needle. A wire is placed through the needle into the vein, and the needle is then removed. Finally, a prosthesis is guided into position in the vein by the wire and the wire is then removed.

The transducer can be attached directly to the support rod without the use of damping material.

## Claims

1. An apparatus for use in the cannulation of blood vessels comprising: a hollow needle (24) with an inner lumen, a sharp distal end which is adapted to be inserted into a blood vessel, and a proximal end which is adapted to be secured to a syringe (28); an elongate stylet (26) which is positioned within the inner lumen of the needle (24), the stylet comprising an elongate support means (40), a transducer (34) attached to the support means at the distal end of the stylet for transmitting and receiving ultrasonic waves through the sharp end of the needle (24) and adapted to generate a signal representing the intensity of the received ultrasonic waves, the transducer (34) having front and back faces; and a syringe (28) which is detachably secured to the proximal end of the needle (24), characterised in that the stylet is spaced from the interior of the needle (24) to facilitate back flow of blood when a blood vessel is pentrated, and by the provision of electrical conductors which are associated with the stylet (26) for transmitting electrical signals to and from the transducer (34) and include a first electrical conductor (42) which extends through the stylet (26) and is electrically connected to the back face of the transducer, and a second electrical conductor (48) which is electrically insulated from the first conductor (42), extends along the outside of the stylet (26) and is electrically connected to the front face of the transducer.

2. An apparatus as claimed in Claim 1, wherein the second electrical conductor (48) contacts the needle (24) whereby the needle forms a part of the conductor.

3. An apparatus as claimed in Claim 1 or 2, wherein the transducer (32) has metal contacts (44, 50) on its faces contacting the electrical conductors (42, 48).

4. An apparatus as claimed in Claim 1, 2 or 3, wherein the transducer (34) is attached to the support means (40) by means (46) which includes an ultrasonic damping material.

## Patentansprüche

1. Vorrichtung zur Verwendung bei der Kanalisation von Blutgefäßen mit einer einen lichten Innenraum, ein in ein Blutgefäß einzuführendes scharfes freies Ende und ein an einer Spritze (28) zu befestigendes dieser nächstgelegenes Ende aufweisenden hohlen Nadel (24); einem im lichten Innenraum der Nadel (24) angeordneten langgestreckten Sondenführer (26), der langgestreckte Halterungsmittel (40) und einen an den Halterungsmitteln am freien Ende des Sondenführers befestigten Wandler (34) mit einer Vorder- und einer Hinterseite zur Übertragung und zum Empfang von Ultraschallwellen durch das scharfe Ende der Nadel (24) sowie zur Erzeugung eines die Intensität der empfangenen Ultraschallwellen repräsentierenden Signals umfaßt; und einer lösbar am ihr nächstliegenden Ende der Nadel (24) befestigten Spritze (28), **dadurch gekennzeichnet**, daß der Sondenführer zur Erleichterung eines Blutrückflusses beim Eindringen in ein Blutgefäß vom Inneren der Nadel (24) beabstandet ist, und daß dem Sondenführer zur Übertragung elektrischer Signale zum und vom Wandler (34) elektrische Leiter zugeordnet sind, die einen ersten durch den Sondenführer (26) verlaufenden und elektrisch mit der Rückseite des Wandlers verbundenen elektrischen Leiter (42) und einen zweiten vom ersten Leiter (42) isolierten, längs der Außenseite des Sondenführers (26) verlaufenden und elektrisch mit der Vorderseite des Wandlers verbundenen elektrischen Leiter (48) enthalten.

2. Vorrichtung nach Anspruch 1, bei welcher der zweite elektrische Leiter (48) mit der Nadel (24) in Kontakt steht, wodurch die Nadel einen Teil des Leiters bildet.

3. Vorrichtung nach Anspruch 1 oder 2, bei welcher der Wandler (42) Metallkontakte (44, 50) auf seinen mit den elektrischen Leitern (42, 48) in Kontakt stehenden Flächen besitzt.

4. Vorrichtung nach Anspruch 1, 2 oder 3, bei welcher der Wandler (34) durch ein Ultraschalldämpfungsmaterial enthaltende Mittel (46) an den Halterungsmitteln (40) befestigt ist.

## Revendications

1. Dispositif destiné à être utilisé dans la canalisation de vaisseaux sanguins comprenant : une aiguille creuse (24) avec une lumière interne, une extrémité distale pointue qui est adaptée à être insérée dans un vaisseau sanguin, et une extrémité proximale qui est adaptée à être fixée à une seringue (28); un stylet allongé (26) qui est positionné au sein de la lumière interne de l'aiguille (24), le stylet comprenant un moyen de support allongé (40), un transducteur (34) fixé au moyen de support à l'extrémité distale du stylet pour transmettre et recevoir des ondes ultrasonores via l'extrémité pointue de l'aiguille (24) et adapté à produire un signal représentant l'intensité des ondes ultrasonores reçues, le transducteur (34) comportant des faces avant et arrière; et une seringue (28) qui est fixée avec possibilité d'enlèvement à l'extrémité proximale de l'aiguille (24), caractérisé en ce que le sty[et est espacé de l'intérieur de l'aiguille (24) afin de faciliter le reflux du sang lorsqu'un vaisseau sanguin est percé, et par l'utilisation de conducteurs électriques qui sont associés au stylet (26) pour transmettre des signaux électriques vers et depuis le transducteur (34) et comprennent un premier conducteur électrique (42) qui s'étend à travers le stylet (26) et est relié électriquement à la face arrière du transducteur, et un second conducteur électrique (48) qui est isolé électriquement du premier conducteur (42), s'étend le long de l'extérieur du stylet (26) et est relié électriquement à la face avant du transducteur.

2. Dispositif selon la revendication 1, dans lequel le second conducteur électrique (48) contacte l'aiguille (24) de sorte que l'aiguille constitue une partie du conducteur.

3. Dispositif selon la revendication 1 ou 2, dans lequel le transducteur (34) comporte des contacts métalliques (44, 50) sur ses faces contactant les conducteurs électriques (42, 48).

4. Dispositif selon la revendication 1, 2 ou 3, dans lequel le transducteur (34) est fixé au moyen de support (40) par un moyen (46) qui comprend un matériau d'amortissement à ultrasons.
